# EUROPEAN PATENT SPECIFICATION

(11) **EP 0 736 292 B1**
(45) Date of publication and mention of the grant of the patent: **19.12.2001**
(21) Application number: 96302382.5
(22) Date of filing: 03.04.1996
(51) Int. Cl.: A61F 2/38, A61B 17/16

(54) **Prosthetic patella implant of the knee joint**
Prosthetischer Patella-Implant des Kniegelenkes
Implant prosthétique patellaire du genou

(30) Priority: 07.04.1995 GB 9507285
(43) Date of publication of application: 09.10.1996
(73) Proprietor: Mendes, David G., 34 987 Haifa (IL); Beer, Ruth, 34 987 Haifa (IL)
(72) Inventor: Gruebel Lee, David Mark, Farnham, Surrey (GB); Mendes, David G., 34 987 Haifa (IL); Beer, Ruth, 34 987 Haifa (IL)
(74) Representative: Musker, David Charles

(56) References cited:
- EP-A- 0 438 918
- EP-A- 0 676 182
- WO-A-93/00871
- WO-A-94/10914
- US-A- 4 964 867
- US-A- 5 021 061

## Description

The present invention relates to surgical implants or prosthetics and, more particularly, to a design for an artificial patella, or knee cap, and a tool for its installation.

Joint replacement is becoming increasingly widespread. One of the most widely practiced joint replacement involves the knee joint. In many cases, the replacement of the knee joint with a prosthetic also involves the replacement of a portion of the patella with a prosthetic.

Partial replacement of the patella is widely used in the surgical replacement of a damaged portion of the knee joint. However, it is known that, in a significant percentage of the cases, the patella implant typically fails after five to fifteen years. One of the typically occurring failures is near or at the periphery of the circular of elliptical patella implant, where the thickness of the patella implant material, typically high molecular weight high density polyethylene (HDPE), is at its smallest. A failing patella could lead to significant pain in the patient and typically requires a second operation to replace the failed patella implant. Such a failure of a HDPE patella implant is disclosed in "Wear and Deformation of Patella Components in Total Knee Arthroplasty" by H-P Hso and P.S. Walker from Clinical Orthopedics and Related Research 246 pages 260-265.

There is thus a widely recognized need for, and it would be highly advantageous to have, a patella implant which will be more durable than those currently known and which will be significantly less prone to damage due to insufficient thickness, typically about its periphery.

According to the present invention there is provided a patella implant adapted to structurally fit a remaining part of a natural patella comprising an upper surface, for sliding over a femoral articulating member, and an undersurface, for fixation to the natural patella, characterised in that the distance between said upper surface and said under surface defines a circumferential facet having a height of at least 6mm and said under surface is substantially concave for fixation to a convexly sectioned natural patella, thereby reserving a maximum amount of healthy natural tissue and minimising wear of the circumference of the implant.

According to the present invention, there is further provided a reamer for use in preparing a natural patella to accept a patella implant having a substantially concave undersurface, comprising: (a) a concave rotatable reaming member, the concavity of said rotatable member being substantially equal to the concavity of the undersurface, of the patella implant; and (b) a bit protruding from said concave rotatable reaming member for drilling a hole in the natural patella.

According to further features in preferred embodiments of the invention described below, the patella implant has a minimum thickness, typically at its periphery, of not less than about 6 or 8mm.

The present invention relates to a special design for surgical replacement of part of the natural patella (knee cap) with a prosthetic part. The invention has been found particularly useful for patella replacement combined with replacement of all articulating parts of the knee joint but is also useful for replacement of the patella only.

An object of the present invention is to provide a design and a surgical tool for fixing the patella implant to the remaining portion of the natural patella which will enhance the strength and durability of the patella implant in vivo.

According to the present invention there is provided a design which will enable the manufacture of a HDPE patella implant with an overall thickness of not less than about 8mm. This thickness is considered in the scientific literature as an optimal thickness for a high molecular weight high density polyethylene (HDPE) patella implant for use in an average person weighing 60-70 kg, for preventing high stresses within the material. Smaller thicknesses are to be used in smaller patients.

The use of the augmented minimum thickness eliminates one of the main causes of failure of patella implants and enhances the durability of the implant.

A patella implant according to the present invention is disc-shaped, typically either circular or elliptical, and has a typical diameter of about 25 to about 40 mm which roughly matches the diameter of the bony patella, but may by of smaller diameter. The disc like implant is characterized in that it has a concave undersurface.

The implant also has an upper surface which may have any suitable shape, including, but not limited to, concave and flat, but preferably, is substantially convex. In the case of a convex upper surface, the convexity may, but need not, conform to the concavity of the undersurface.

To provide the required optimal thickness of the patella implant, the natural patella is cut, reamed and trimmed in such a manner as to remove a total of up to about 8 mm or more from the natural bone to leave a convex shape which complements the concave shape of the undersurface of the patella implant.

The concave undersurface of the patella implant fits the appropriately reamed remaining portion of the natural patella. The upper surface articulates with the articulating femoral member, typically a groove, and is shaped to fit the corresponding articulating portion of the femoral component of the total knee implant. when the articulating femoral member is a groove, the upper surface of the implant is typically

Substantially convex. Where the upper surface is convex the convexity of the upper surface and the concavity of the undersurface of a patella implant according to the present invention do not necessarily conform to each other and may be independently varied to accommodate the specific design of the femoral groove, or its equivalent, and the convexity of the prepared natural patella.

### BRIEF DESCRIPTION OF THE DRAWINGS

The invention is herein described, by way of example only, with reference to the accompanying drawings, wherein:
FIG. 1 is a perspective view of a typically artificial knee joint;
FIG. 2 is a side view of an artificial knee joint as in Figure 1 showing the patella, quadriceps tendon and patella tendon;
FIG. 3 is a cross sectional view of a conventional prior art patella implant;
FIG. 4 is a back view of the patella implant of Figure 3;
FIG. 5 is a front view of the patella implant of Figure 3;
FIG. 6 is a cross sectional view of one embodiment of a patella implant according to the present invention;
FIG. 7 is a back view of the patella implant of Figure 6;
FIG. 8 is a front view of the patella implant of Figure 6;
FIG. 9 is a cross sectional view of another embodiment of a patella implant according to the present invention;
FIG. 10 is a back view of the patella implant of Figure 9;
FIG. 11 is a front view of the patella implant of Figure 9;
FIG. 12 is a back view of a patella implant showing a pair of fixation members;
FIG. 13 is a back view of a patella implant showing four fixation members;
FIG. 14 is a side view of a reamer which may be used to prepare a natural patella for acceptance of a patella implant according to the present invention.
FIG. 15 cross sectional view of an onlay patella implant
FIG. 16 cross sectional view of an inlay patella implant showing circumferential facetsin the shape of acone.
FIG. 17 partial cross sectional view of Fig 16 showing angle of circumferential facet.

### DESCRIPTION OF THE PREFERRED EMBODIMENTS

The present invention is of a patella implant which can be used in the surgical repair of knee joints.

The principles and operation of a patella implant according to the present invention may be better understood with reference to the drawings and the accompanying description.

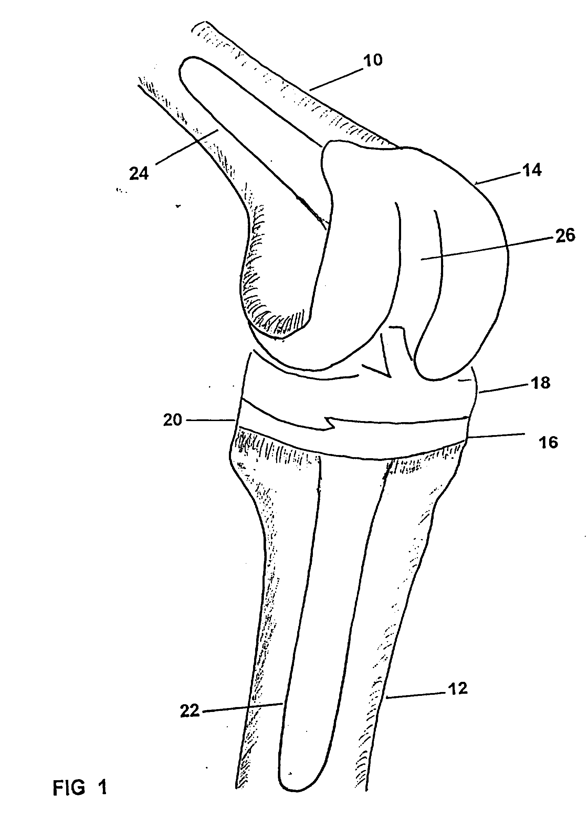

Referring now to the drawings, Figure 1 illustrates a typical knee joint prosthetic. The knee joint is formed between the lower end of the femur 10 and the upper end of the tibia 12. In a total knee replacement, the lower end of femur 10 is replaced with a femoral prosthetic component 14 while the upper end of tibia 12 is replaced with a tibial prosthetic component 16.

Tibial prosthetic component **16** is typically made up of a plastic upper plate **18** and a metal back **20.** A tibial anchorage stem **22** connected to metal plate **18** is typically used to anchor tibial prosthetic component **16** into tibia **12.**

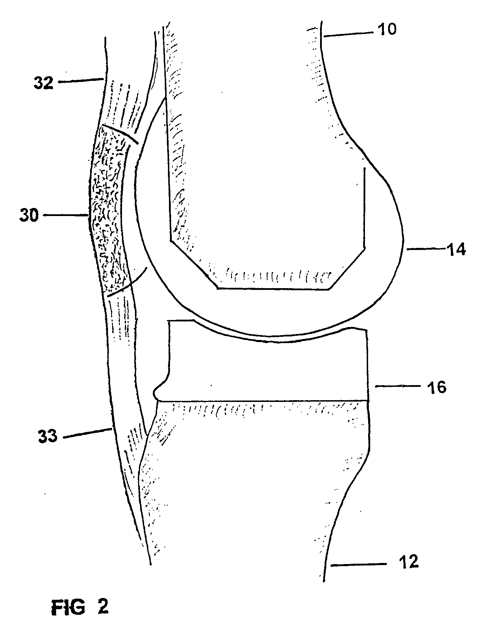

Femoral prosthetic component **14** is typically made of metal and is anchored into femur **10** with a femoral anchorage stem **24.** The face of femoral prosthetic component **14** which contacts tibial prosthetic component **16** is typically shaped to mimic the natural knee to include a groove **26.** It is on groove **26,** or its equivalent, that the patella slides, as can be best be seen in Figure 2.

Figure 2 illustrates a total knee implant including the. replacement of the articulating portion (the portion facing the knee) of the patella.A s Figure 2 illustrates, the patella (knee cap) **30** is a disc-shaped member which is connected to the quadriceps tendon **32** and to the patella tendon **33** and is slidable over the lower end of femur **10**. The portion of patella **30** facing the knee (the patella implant) is typically, but not necessarily, convex and is dimensioned to slidably engage the corresponding portion of femoral prosthetic component **14,** typically groove **26** (Figure 1). The portion of patella **30** away from the knee (the remaining natural patella) is connected to quadriceps tendon **32** and patella tendon **33.** Quadriceps tendon **32** is connected to the quadriceps muscle which is, in turn, attached to femur **10.** Patella tendon **33** is connected to tibia **12.** In this way patella **30** slides over the knee joint during flexion and extension of the joint. The presence of patella **30** facilitates the sliding of quadriceps tendon **32** and further enhances its mechanical efficiency.

To surgically repair a damaged patella what is done is to remove a portion of the convex articulating surface (the surface facing the knee joint) of the natural patella leaving the connection between the natural patella and the muscle intact. The removal is typically effected using a vibrating saw or similar instrument.

Once a portion of the patella has been removed, a prosthetic, or implant, may be fixed to the remaining portion of the natural patella by some suitable means. The implant is shaped to slidably fit within the groove, of equivalent, of the corresponding natural or prosthetic lower end of the femur, depending on whether the natural lower femur is to remain or be replaced, respectively. Attachment of the implant to the natural patella may be effected with adhesives, cements or other bonding materials and/or through use of pegs, as described in more detail below.

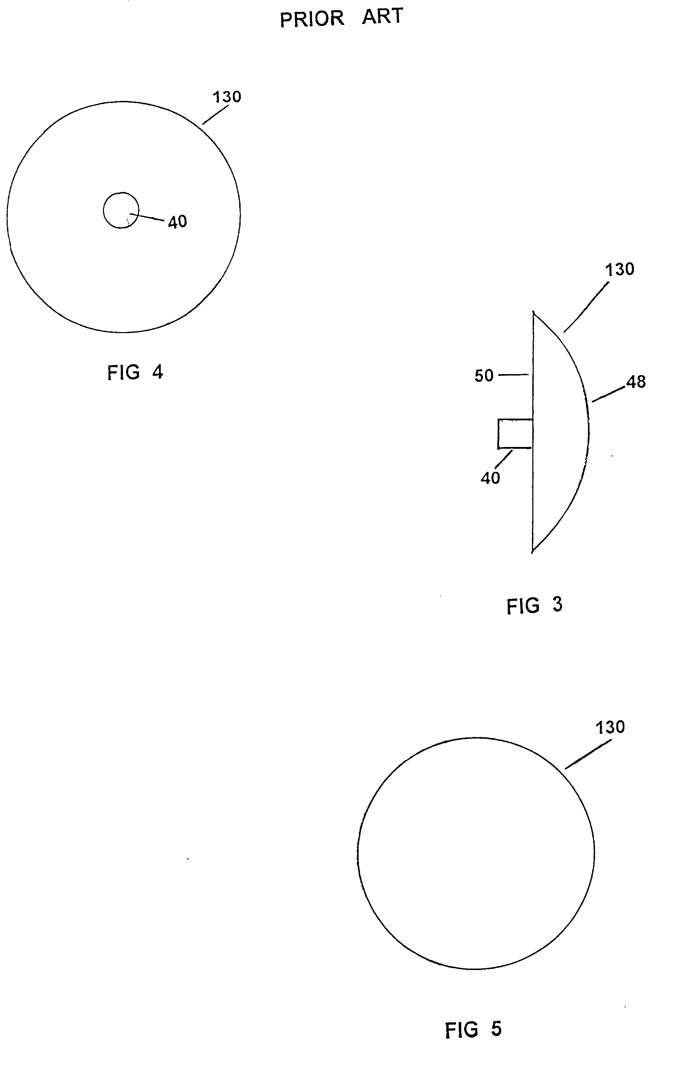

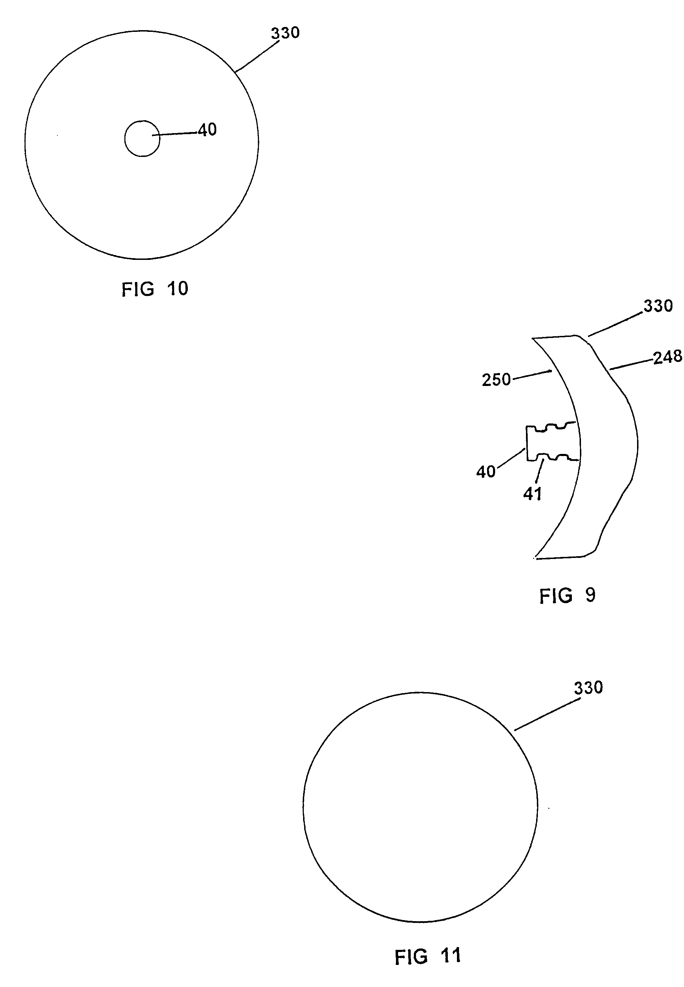

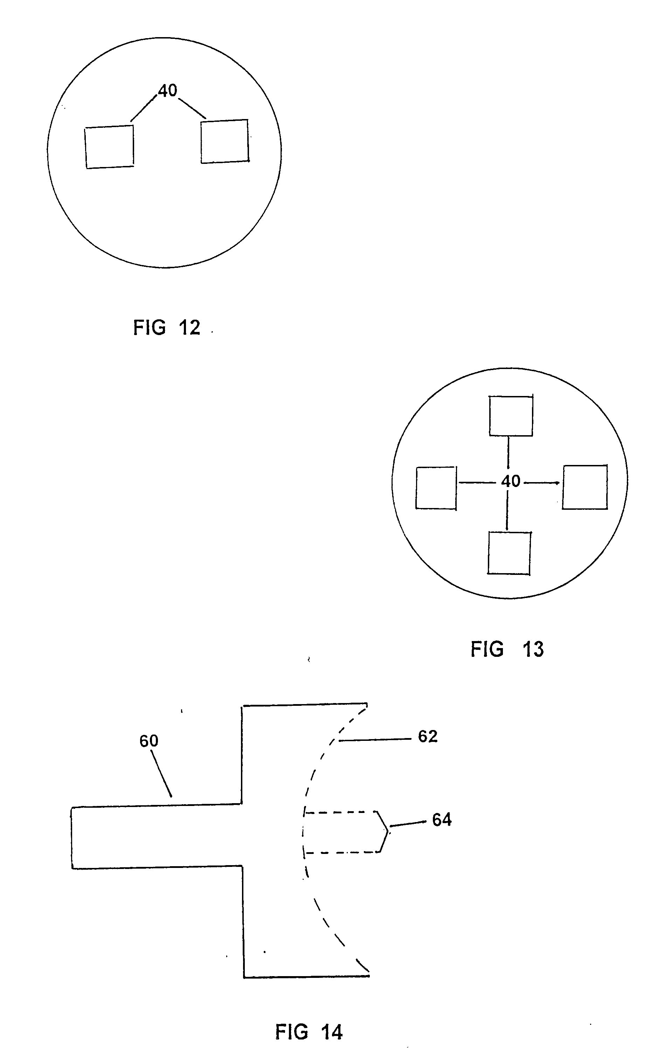

A typical conventional patella implant is shown in side, back and front views in Figures 3, 4 and 5, respectively. Patella implants are typically circular in back and front views (Figures 4 and 5) but may also be elliptical (as can be seen in Figures 10 and 11) or may assume other shapes. Furthermore, patella implants typically include one or more protrusions, or pegs, **40** which are inserted into the natural patella and help firmly fix, typically with the addition of suitable adhesives, cements or other bonding materials, the implant and the remaining portion of the natural patella. Protrusions **40** may be integrally formed with the implant or may be connected to it in some suitable fashion, as by screwing, for example. The one or more protrusions **40** may be of various shapes, including, but not limited to square or rectangular (see Figures 12 and 13). Preferably, protrusions **40** are circular in cross-section, or cylindrical (Figures 3, 6 and 9). Most preferably, the implant includes a single central circular protrusion **40** (Figures 3, 6 and 9).

As can best be seen in the side view of Figure 3, a conventional patella implant **130** includes a surface (hereinafter "upper surface") **48** of any suitable design, typically, but not necessarily, substantially convex, and a planar, i.e., flat, undersurface **50.** To surgically install such an implant, the natural patella is cut in planar, or flat, fashion and the implant is bonded, or otherwise fixed, (with or without the help of one or more pegs **40)** to the newly cut face of the natural patella.

The geometry of the conventional patella implant **130** is such that the periphery of implant **130** is very thin compared to its central portion. It has been determined that, because of this geometry, under the tremendous stresses to which the implant is exposed during normal use, failure of the implant frequently takes place mostly near and at the periphery. Failure typically results from wear, creep and fatigue cracks of the high molecular weight high density polyethylene from which the implant is made.

A patella implant according to the present invention overcomes these difficulties by providing a minimum thickness of the implant, including near and at its periphery, which is significantly larger than that available with the presently known implants described above. The increased thickness, particularly near and at the periphery, is accomplished by providing a substantially concave undersurface for fixation to the remaining portion of the natural patella.

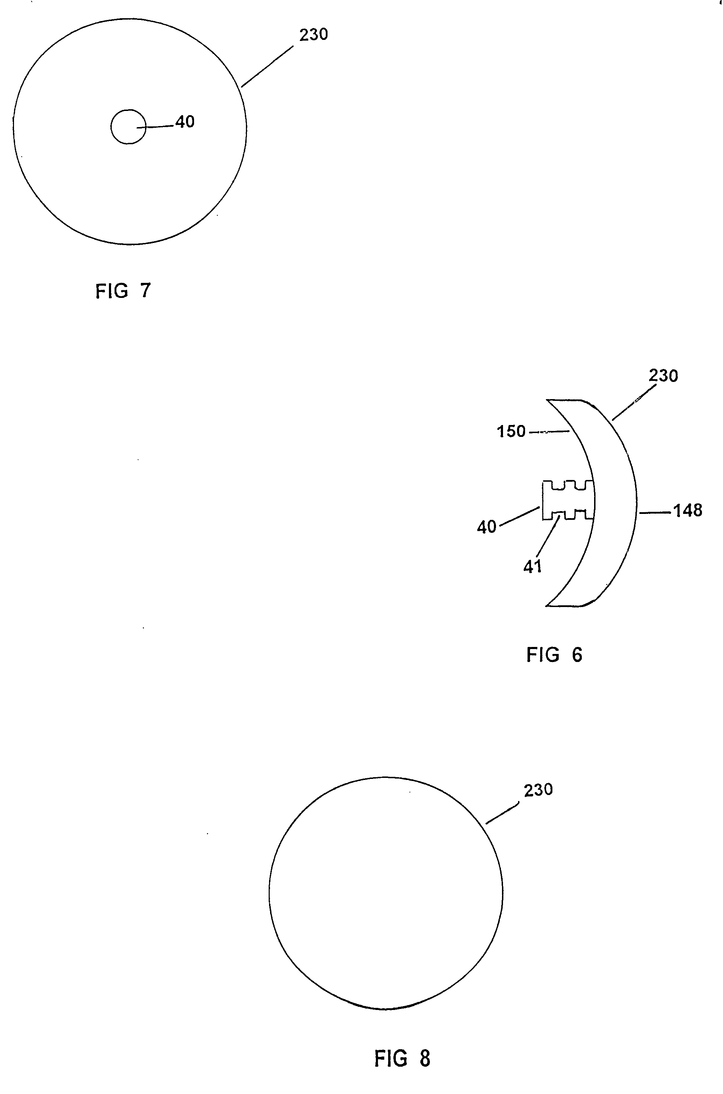

Thus, with reference to Figures 6 and 9, a patella implant, **230** or **330,** according to the present invention includes an upper surface, **148** or **248,** which is typically, but not necessarily, substantially convex, for sliding over femoral groove **26,** or its equivalent (Figure 1). Upper surface, **148** or **248,** can have any suitable shape designed to slidably engage groove **26** of femoral prosthetic component **14.** Two illustrative examples of upper surface geometries, both substantially convex, are shown in Figures 6 and 9 wherein the substantially convex surface has a monotonically changing slope and a slope which features an inflection, respectively. The surface finish of the upper surface is preferably prepared in accordance with the conventional requirements of the Food and Drug Administration (FDA).

Patella implant, **230** or **330,** is characterized in that it further includes a substantially concave undersurface, **150** or **250,** for fixation to the remaining portion of the natural patella. The concavity of undersurface, **150** or **250,** in contrast with the planar undersurface **50** of conventional patella implants (Figure 3) makes it possible to have an implant with significantly larger minimum thickness, particularly around its periphery.

The diameter of the patella implant should approximately correspond to the size of the remaining natural patella. All portions of the patella implant, including the periphery, should preferably have thicknesses of not less than about 8 mm in the case of normal-sized patients and not less than about 6 mm in the case of small patients, where the implant material is high molecular weight high density polyethylene (HDPE). It will be readily appreciated that when different materials are used for the implant, including, but not limited to, other plastics, various metals, ceramics or composites, a different optimal minimum thickness will be appropriate.

As described above, the concave surface should preferably include one or more pegs **40** which are preferably either cylindrical or rectangular, but which can be any other geometrical shape. Pegs **40** may be of any suitable size, preferably at least 2 mm. Most preferably, pegs **40** should extend beyond the undersurface of the implant so that their distal end is substantially flush with the outer rim of the undersurface, typically about 6 mm. The diameter, or equivalent diameter, of pegs **40** should preferably be at least 5 mm, most preferably on the order of 10 mm.

Preferably, pegs **40** are formed with circumferential depressions **41** (Figures 6 and 9) which help improve the bonding and anchorage by providing enhanced friction and further providing space in which adhesives and the like can accumulate.

Pegs **40** are dimensioned to fit into holes prepared in the natural patella following the reaming of the bony patella during the preparation of the bony patella. Pegs **40** and the concave undersurface of the patella implant are prepared to fix to the natural patella either by means of suitable bonding materials, such as, for example, an acrylic cement, or by special materials fit for bone ingrowth, such as, for example, metallic or ceramic coatings.

According to a preferred embodiment of the present invention the patella implant is constructed of high molecular weight high density polyethylene, although other biocompatible materials, such as various metals, ceramics, plastics or composites, may be used. For convenience of the user the implants may be marketed in several overall diameters, for example, 25 mm with increments of 3-4 mm, and in several optional thicknesses, for example, 8 mm with increments of 1-2 mm. Of course, for small patients, implants with thicknesses of less than 8 mm will be available.

As will readily be appreciated, when an implant according to the present invention is made of a material other than HDPE, such as, for example, a metal, the recommended thickness may be different. However, the concepts of shape and anchorage described herein are the same.

The concave underside of the implant can have a single radius of curvature or can feature a flattened central portion. Other shapes are also possible and are intended to be included within the scope of the present invention. Other shapes may be used which will enhance the bonding of the implant to the natural patella. To further enhance the bonding and fixation of the implant to the bone, it may be desirable to use a number of pegs **40** of various shapes (for example, Figures 12 and 13).

To anchor pegs **40** in the natural patella, a single central or nearly central hole and/or one or more non-central holes are prepared in the remaining portion of the natural patella. Fixation of the prosthetic patella can be effected by means of a bonding material or other chemical, physical or biological adhesives and by biological reactions, such as bone ingrowth into the surface, preferably using the pegs which fit into their respective holes in the prepared bone surface. Alternatively or additionally, fixation may be effected by means of press fitting or other fixation techniques.

To prepare the natural patella bone to accept an implant according to the present invention, it is necessary to cut the bone so that it takes on the shape of the concave undersurface of the implant. The surgical preparation of the natural patella can be achieved using a special tool which cuts the bony patella to precisely the desired shape with minimal interference of its blood supply form the surrounding tissues.

The special tool is a concave surgical reamer 60 (Figure 14) which can be powered electrically, pneumatically, mechanically, manually, and the like. Reamer 60 can be used to remove an appropriate amount of bone in order to create a convex surface of cortical and/or cancellous bone of the bony patella which accurately fits the concave undersurface of a patella implant according to the present invention.

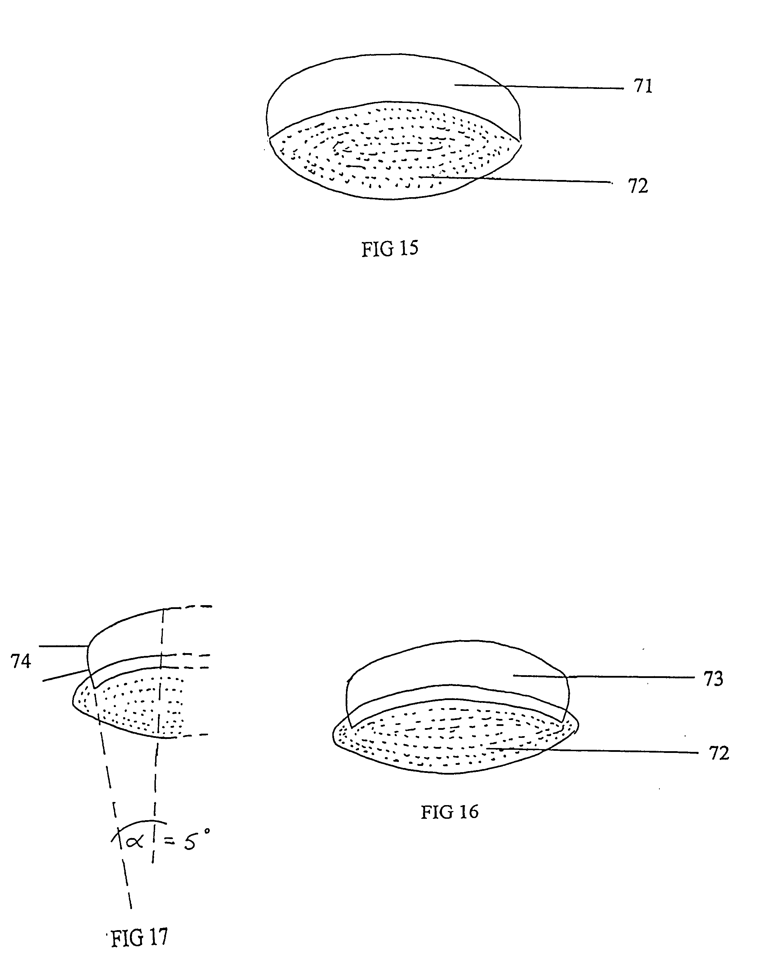

Reamer 60 includes a concave rotatable reaming member 62 whose concavity is substantially equal to the concavity of the patella implant undersurface. Preferably, reamer 60 further includes a bit 64 which protrudes from concave rotatable reaming member 62 and which is used to simultaneously drill a hole in the natural patella which will accommodate a single central peg extending from the undersurface of the implant.
While the invention has been described with respect to a limited number of embodiments, it will be appreciated that many variations, modifications and other applications of the invention may be made, in particular it will be appreciated that the present invention may be used in a variety of surgical applications such as an onlay of implant 71 on bone 72 (FIG 15) or as an inlay implant when implant 73 is partly intruding into bone 72 (FIG 16). It is appreciated that configuration of the circumferential facet 74 of the implant has many variations including but not limited to cylindrical or conical with the angle of the cone of about 5° (FIG 17) which allows a press fit of the inlay implant into bone.

## Claims

1. A patella implant (230; 330) adapted to structurally fit a remaining part of a natural patella (30) comprising an upper surface (48; 148; 248), for sliding over a femoral articulating member, and an undersurface (50; 150; 250), for fixation to the natural patella (30), **characterised in that** the distance between said upper surface (148; 248) and said under surface (150; 250) defines a circumferential facet having a height of at least 6mm and said under surface (150; 250) is substantially concave for fixation to a convexly sectioned natural patella (30), thereby reserving a maximum amount of healthy natural tissue and minimising wear of the circumference of the implant (130; 230).

2. A patella implant (130; 230) according to claim 1 in which said implant (130; 230) is adapted for specific geometries of femoral articulating member and said convexly sectioned natural patella (30), wherein said distance between said upper surface (148; 248) and undersurface (150; 250) is variable and not less than a minimal value, and said facet having a height of said minimal value.

3. A patella implant (130; 230) according to claim 1 or 2, in which the implant (130; 230) is made of plastic.

4. A patella implant (130; 230) according to claim 3, in which the implant (130; 230) is made of high molecular weight high density polyethylene.

5. A patella implant (130; 230) according to claim 1 or 2, in which said implant (130; 230) is made from a metal or a ceramic or a composite material.

6. A patella implant (130; 230) according to any preceding claim, in which said height of said facet is not less than 8mm.

7. A patella implant (130; 230) according to any preceding claim, in which the implant (130; 230) is substantially circular in plan view.

8. A patella implant (130; 230) according to any one of claims 1 to 7, in which the implant (130; 230) is substantially elliptical in plan view.

9. A patella implant (130; 230) according to claim 7, in which said upper surface (148; 248) and said undersurface (150; 250) comprise a structure of a dome.

10. A patella implant (130; 230) according to claim 7, in which said upper surface (148; 248) is substantially convex and includes an inflection.

11. A patella implant (130; 230) according to claim 1, in which said circumferential facet comprises a cone of approximately 5° angle.

12. A patella implant (130; 230) as in any preceding claim, wherein said upper surface (148; 248) is substantially convex and features a monotonically changing slope.

13. A patella implant (130; 230) according to any preceding claim, in which said undersurface (150; 250) comprises at least one protrusion (40).

14. A patella implant (130; 230) according to claim 13, in which said protrusion (40) is integrally formed with the implant (130; 230).

15. A patella implant (130; 230) according to claim 13, in which said protrusion (40) is connected to the implant (130; 230).

16. A patella implant (130; 230) according to any one of claims 13 to 15, in which at least one protrusion (40) is substantially circular in cross-section.

17. A patella implant (130; 230) according to any one of claims 13 to 16, in which at least one protrusion (40) extends at least 2 mm beyond said undersurface (150; 250) of the implant (130; 230).

18. A patella implant (130; 230) according to any one of claims 13 to 17, in which at least one protrusion (40) has a diameter of at least 8 mm.

19. A patella implant (130; 230) as claimed in any one of claims 13 to 18 in which said protrusion (40) includes circumferential depressions (41).

20. A patella implant (130; 230) according to claim 1, in which said undersurface (150; 250) comprises at least one protrusion (40) of at least 8mm diameter and which extends at least 2mm beyond said undersurface (150; 250) of the implant and said convexly sectioned natural patella (30) comprises at least one depression for mounting said protrusion (40).

21. A reamer (60) for use in preparing a natural patella (30) to accept a patella implant (230; 330) having a substantially concave undersurface (150; 250), comprising:
(a) a concave rotatable reaming member (62), the concavity of said rotatable member (62) being substantially equal to the concavity of the undersurface (150; 250), of the patella implant (230; 330); and
(b) a bit (64) protruding from said concave rotatable reaming member (62) for drilling a hole in the natural patella (30).

## Patentansprüche

1. Kniescheibenimplantat (230; 330), so eingerichtet, daß es einem verbliebenen Teil einer natürlichen Kniescheibe (30) strukturell angepaßt ist, umfassend
eine obere Oberfläche (48; 148; 248) zum Gleiten über ein zum Oberschenkelknochen gehörendes Gelenkglied und
eine untere Oberfläche (50; 150; 250) zur Befestigung an der natürlichen Kniescheibe (30),
**dadurch gekennzeichnet, daß** der Abstand zwischen der oberen Oberfläche (148; 248) und der unteren Oberfläche (150; 250) eine Umfangsfacette mit einer Höhe von mindestens 6 mm definiert und daß die untere Oberfläche (150; 250) zur Befestigung an einer konvex geschnittenen natürlichen Kniescheibe (30) im wesentlichen konkav ist, wodurch ein maximaler Anteil des gesunden natürlichen Gewebes erhalten bleibt und der Verschleiß am Umfang des Implantats (130; 230) minimiert wird.

2. Implantat nach Anspruch 1, das für spezifische Geometrien von zum Oberschenkelknochen gehörigen Gelenkgliedern und konvex geschnittenen natürlichen Kniescheiben (30) eingerichtet ist, wobei der Abstand zwischen der oberen Oberfläche (148; 248) und der unteren Oberfläche (150; 250) variabel und nicht kleiner als ein minimaler Wert ist und die Facette eine Höhe gemäß dieses minimalen Wertes aufweist.

3. Implantat nach Anspruch 1 oder 2, das aus Plastik hergestellt ist.

4. Implantat nach Anspruch 3, das aus Polyäthylen mit hohem Molekulargewicht und hoher Dichte hergestellt ist.

5. Implantat nach Anspruch 1 oder 2, das aus einem Metall, einem keramischen oder einem zusammengesetzten Material hergestellt ist.

6. Implantat nach einem der vorhergehenden Ansprüche, wobei die Höhe der Facette nicht weniger als 8 mm beträgt.

7. Implantat nach einem der vorhergehenden Ansprüche, das in Draufsicht im wesentlichen kreisförmig ist.

8. Implantat nach einem der Ansprüche 1 bis 7, das in Draufsicht im wesentlichen elliptisch ist.

9. Implantat nach Anspruch 7, wobei die obere Oberfläche (148; 248) und die untere Oberfläche (150; 250) die Struktur einer Kuppel aufweisen.

10. Implantat nach Anspruch 7, wobei die obere Oberfläche (148; 248) im wesentlichen konvex ist und eine Wendung beinhaltet.

11. Implantat nach Anspruch 1, wobei die Umfangsfacette einen Kegel mit einem Winkel von ungefähr 5° umfaßt.

12. Implantat nach einem der vorhergehenden Ansprüche, wobei die obere Oberfläche (148; 248) im wesentlichen konvex ist und eine sich monoton ändernde Neigung aufweist.

13. Implantat nach einem der vorhergehenden Ansprüche, wobei die untere Oberfläche (150; 250) mindestens eine Auskragung (40) aufweist.

14. Implantat nach Anspruch 13, wobei die Auskragung (40) mit dem Implantat (130; 230) integral ausgeformt ist.

15. Implantat nach Anspruch 13, wobei die Auskragung (40) mit dem Implantat (130; 230) verbunden ist.

16. Implantat nach einem der Ansprüche 13 bis 15, wobei mindestens eine Auskragung (40) im Querschnitt im wesentlichen kreisförmig ist.

17. Implantat nach einem der Ansprüche 13 bis 16, wobei mindestens eine Auskragung (40) mindestens 2 mm über die untere Oberfläche (150; 250) des Implantats (130; 230) hervorsteht.

18. Implantat nach einem der Ansprüche 13 bis 17, wobei mindestens eine Auskragung (40) einen Durchmesser von mindestens 8 mm aufweist.

19. Implantat nach einem der Ansprüche 13 bis 18, wobei die Auskragung (40) an ihrem Umfang Vertiefungen (41) beinhaltet.

20. Implantat nach Anspruch 1, wobei die untere Oberfläche (150; 250) mindestens eine Auskragung (40) mit einem Durchmesser von mindestens 8 mm umfaßt, die mindestens 2 mm über die untere Oberfläche (150; 250) des Implantats hervorsteht, und wobei die konvex geschnittene natürliche Kniescheibe (30) mindestens eine Vertiefung zum Aufnehmen der Auskragung (40) umfaßt.

21. Reibahle (60), geeignet zum Vorbereiten einer natürlichen Kniescheibe (30) zum Aufnehmen eines Kniescheibenimplantats (230; 330) mit einer im wesentlichen konkaven unteren Oberfläche (150; 250), umfassend:
(a) ein konkaves drehbares Reibahlglied (62), dessen Konkavität im wesentlichen gleich der Konkavität der unteren Oberfläche (150; 250) des Kniescheibenimplantats (230; 330) ist; und
(b) eine von dem konkaven drehbaren Reibahlglied (62) hervorstehende Bohrerspitze (64), um ein Loch in die natürliche Kniescheibe (30) zu bohren.

## Revendications

1. Implant patellaire (230; 330) prévu pour s'adapter structuralement à une partie restante d'une patelle naturelle (30), comprenant une surface supérieure (48; 148; 248) destinée à coulisser sur un élément articulaire fémoral, et une surface de dessous (50; 150; 250) destinée à être fixée à la patelle naturelle (30), **caractérisé en ce que** la distance entre ladite surface supérieure (148; 248) et ladite surface de dessous (150; 250) définit une facette circonférentielle ayant une hauteur d'au moins 6 mm, et **en ce que** ladite surface de dessous (150; 250) est sensiblement concave pour être fixée à une patelle naturelle (30) sectionnée de manière convexe, pour ainsi conserver une quantité maximale de tissu naturel sain et réduire au minimum une usure de la circonférence de l'implant (130; 230).

2. Implant patellaire (130; 230) selon la revendication 1, dans lequel ledit implant (130; 230) est adapté à des géométries spécifiques dudit élément articulaire fémoral et de ladite patelle naturelle (30) sectionnée de manière convexe, ladite distance entre ladite surface supérieure (148; 248) et ladite surface de dessous (150; 250) étant variable et non inférieure à une valeur minimale, et ladite facette ayant une hauteur égale à ladite valeur minimale.

3. Implant patellaire (130; 230) selon la revendication 1 ou 2, dans lequel l'implant (130; 230) est formé d'une matière plastique.

4. Implant patellaire (130; 230) selon la revendication 3, dans lequel l'implant (130; 230) est formé d'un polyéthylène à haute densité et à masse moléculaire élevée.

5. Implant patellaire (130; 230) selon la revendication 1 ou 2, dans lequel ledit implant (130; 230) est réalisé à partir d'un métal, d'une céramique ou d'un matériau composite.

6. Implant patellaire (130; 230) selon l'une quelconque des revendications précédentes, dans lequel ladite hauteur de ladite facette n'est pas inférieure à 8 mm.

7. Implant patellaire (130; 230) selon l'une quelconque des revendications précédentes, dans lequel l'implant (130; 230) est sensiblement circulaire en vue en plan.

8. Implant patellaire (130; 230) selon l'une quelconque des revendications 1 à 7, dans lequel l'implant (130; 230) est sensiblement elliptique en vue en plan.

9. Implant patellaire (130; 230) selon la revendication 7, dans lequel ladite surface supérieure (148; 248) et ladite surface de dessous (150; 250) comprennent une structure en dôme.

10. Implant patellaire (130; 230) selon la revendication 7, dans lequel ladite surface supérieure (148; 248) est sensiblement convexe et comprend une inflexion.

11. Implant patellaire (130; 230) selon la revendication 1, dans lequel ladite facette circonférentielle comprend un cône ayant un angle d'approximativement 5°.

12. Implant patellaire (130; 230) selon l'une quelconque des revendications précédentes, dans lequel ladite surface supérieure (148; 248) est sensiblement convexe et présente une inclinaison qui varie de manière monotone.

13. Implant patellaire (130; 230) selon l'une quelconque des revendications précédentes, dans lequel ladite surface de dessous (150; 250) comprend au moins une partie saillante (40).

14. Implant patellaire (130; 230) selon la revendication 13, dans lequel ladite partie saillante (40) est formée solidairement avec l'implant (130; 230).

15. Implant patellaire (130; 230) selon la revendication 13, dans lequel ladite partie saillante (40) est reliée à l'implant (130; 230).

16. Implant patellaire (130; 230) selon .l'une quelconque des revendications 13 à 15, dans lequel une partie saillante (40) au moins a une section transversale sensiblement circulaire.

17. Implant patellaire (130; 230) selon l'une quelconque des revendications 13 à 16, dans lequel une partie saillante (40) au moins s'étend sur au moins 2 mm au-delà de ladite surface de dessous (150; 250) de l'implant (130; 230).

18. Implant patellaire (130; 230) selon l'une quelconque des revendications 13 à 17, dans lequel une partie saillante (40) au moins a un diamètre d'au moins 8 mm.

19. Implant patellaire (130; 230) selon l'une quelconque des revendications 13 à 18, dans lequel ladite partie saillante (40) comprend des creux circonférentiels (41).

20. Implant patellaire (130; 230) selon la revendication 1, dans lequel ladite surface de dessous (150; 250) comprend au moins une partie saillante (40) d'au moins 8 mm de diamètre et qui s'étend sur au moins 2 mm au-delà de ladite surface de dessous (150; 250) de l'implant, et ladite patelle naturelle (30) sectionnée de manière convexe comprend au moins un creux permettant le montage de ladite partie saillante (40).

21. Alésoir (60) destiné à servir à préparer une patelle naturelle (30) à recevoir un implant patellaire (230; 330) comportant une surface de dessous sensiblement concave (150; 250), comprenant :
(a) un élément aléseur rotatif concave (62), la concavité dudit élément rotatif (62) étant sensiblement égale à la concavité de la surface de dessous (150; 250) de l'implant patellaire (230; 330); et
(b) une mèche (64) dépassant dudit élément aléseur rotatif concave (62) pour forer un trou dans la patelle naturelle (30).
